# EUROPÄISCHE PATENTSCHRIFT

(11) **EP 2 978 457 B1**
(45) Veröffentlichungstag und Bekanntmachung des Hinweises auf die Patenterteilung: **02.01.2019**
(21) Anmeldenummer: 14718315.6
(22) Anmeldetag: 26.03.2014
(51) Int. Cl.: A61L 2/00, A61L 2/14

(54) **VORRICHTUNG ZUR ERZEUGUNG EINES STERILEN BEREICHES FÜR EINE OPERATION, UNTERSUCHUNG ODER BEHANDLUNG EINES OBJEKTS, INSBESONDERE EINER PERSON**
DEVICE FOR GENERATING A STERILE AREA FOR AN OPERATION, EXAMINATION OR TREATMENT OF AN OBJECT, IN PARTICULAR OF A PERSON
DISPOSITIF PERMETTANT DE CRÉER UNE ZONE STÉRILE EN VUE D'UNE INTERVENTION CHIRURGICALE, D'EXAMINER OU DE TRAITER UN OBJET, EN PARTICULIER UNE PERSONNE

(30) Priorität: 28.03.2013 DE 102013103248
(43) Veröffentlichungstag der Anmeldung: 03.02.2016
(73) Patentinhaber: Mirwald, Adelheid, 81925 München (DE)
(72) Erfinder: RIESS, Guido, 80801 München (DE)
(74) Vertreter: Eder, Christian
(86) Internationale Anmeldenummer: PCT/DE2014/100102
(87) Internationale Veröffentlichungsnummer: WO 2014/154206

(56) Entgegenhaltungen:
- EP-A1- 1 765 044
- EP-A1- 2 160 081
- EP-A1- 2 170 022
- WO-A1-2012/112042
- WO-A1-2013/071922
- DE-A1-102010 011 643
- KR-A- 20120 136 524
- US-A1- 2002 187 066
- US-A1- 2010 296 977

## Beschreibung

Die Erfindung betrifft eine Vorrichtung zur Erzeugung eines sterilen Feldes für eine Operation, Untersuchung oder Behandlung wenigstens eines Teilbereiches eines Objekts, insbesondere einer Person. Derartige Vorrichtungen und Verfahren finden im Sinne der Erfindung in Umgebungen, wie beispielsweise in Krankenhäusern, in welchen aus hygienischen oder gar medizinischen Gründen möglichst sterile (also von lebenden Mikroorganismen einschließlich ihrer Ruhestadien - z.B. Sporen - befreite) Bedingungen hergestellt werden sollen, Anwendung. Zudem sind Anwendungen in der Veterinärmedizin sowie Reinigung oder Sterilisation von Gegenständen denkbar.

Beispielsweise wird in der DE 20 2009 000 537 U1 sowie der DE 20 2008 018 264 U1 vorgeschlagen, Werkstückoberflächen oder Kavitäten von Substraten vor einem Beschichten mittels sogenanntem kaltem, atmosphärischem Plasma zu behandeln, um Verunreinigungen von der Oberfläche zu entfernen und die Oberflächen zu konditionieren und zu aktivieren.

In jüngster Zeit wird zunehmend der Einsatz von Plasma, insbesondere kaltem Plasma, für eine (human-)medizinische Anwendung diskutiert. Beispielsweise werden in Bild der Wissenschaft, Ausgabe 1/2009, Seite 52, ein Bekämpfen von Fußpilz oder die Behandlung von sich leicht infizierenden Brandwunden als potenzielle Einsatzmöglichkeiten genannt.

Bei dieser angedachten Behandlung, wie auch der Behandlung von Werkstückoberflächen, wird, wird eine statisch gesehene punktuelle Anwendung eines Plasmastrahls vorgeschlagen.

Problematisch ist bei einer Plasmasterilisation neben der auftretenden Temperatur das bei der Plasmaerzeugung (beispielsweise durch UV-Strahlung) entstehende Ozon, das für eine Person oder einen Patienten nicht nur unangenehm ist, sondern sogar gesundheitsschädliche Folgen (Reizgas) haben kann.

Weitere Vorrichtungen mit Einsatz von Plasma, insbesondere kaltem Plasma, sind aus der US 2010/296977 A1, der DE 10 2010 011643 A1, der EP 2 170 022 A1 und der KR 2012 0136524 A bekannt.

Der vorliegenden Erfindung liegt daher die Aufgabe zugrunde, eine Vorrichtung zur Erzeugung eines sterilen Bereiches (d.h. mit wenigstens flächiger Ausdehnung) zu schaffen, welche eine Operation, Untersuchung oder Behandlung wenigstens eines Teilbereiches eines Objekts, insbesondere einer Person, auf sichere und einfache Art und Weise ermöglicht, ohne dass die Person einem gesundheitlich gefährlichen Risiko ausgesetzt wird oder ein Gegenstand beschädigt wird.

Diese Aufgabe wird erfindungsgemäß durch eine Vorrichtung mit den Merkmalen des Patentanspruchs 1 gelöst.

Nach der Erfindung wird durch den Einsatz einer Vorrichtung, welche wenigstens einen Plasmagasgenerator umfasst, ein steriler Bereich, insbesondere ein steriles Feld für eine Operation, Untersuchung oder beliebige Behandlung, im Folgenden OP-Feld genannt, erzeugt. Im Sinne der Erfindung stellt ein steriler Bereich, insbesondere ein steriles Feld, einen über einen punktuelle Benutzung hinausgehenden Bereich dar, welcher statisch zu einem beliebigen Zeitpunkt während eines Behandlungszeitraums abgedeckt wird bzw. sterilisierendes Plasma in ausreichender Konzentration aufweist.

Ein bekannter punktueller Einsatz einer sogenannten Plasmafackel stellt dagegen selbst bei einem Überfahren oder Überstreichen einer Linie oder eines kleinen Bereiches mit der Plasmafackel statisch gesehen im Wesentlichen nur eine punktuelle Benetzung dar, bei der andere noch nicht überfahrene Bereiche oder bereits überfahrene Bereiche noch nicht oder nicht mehr von Plasma abgedeckt werden. Eine Behandlung eines über einen im Wesentlichen punktuellen Bereich hinausgehenden Bereiches oder Feldes ist hiermit daher nicht möglich. Zudem wäre die Länge eines derartigen Plasmastrahls zu gering, um eine Vergrößerung der Behandlungsfläche (statt einer Spitze bzw. eines Punktes) durch eine Vergrößerung des Abstandes zum Werkstück zu erreichen.

Die erfindungsgemäße Vorrichtung weist zur Erzeugung des sterilen Bereiches beispielsweise am Rand des Bereiches ein zumindest teilweise umgebendes Gehäuse auf, in welchem vorzugsweise mehrere Plasmageneratoren derart angeordnet sind, dass sie bei Bedarf impulsartig oder kontinuierlich Plasmagas in Richtung zum Inneren des Bereiches emittieren, so dass während einer Behandlung zu jedem Zeitpunkt eine ausreichende Konzentration von sterilisierendem Plasmagas vorhanden ist. Die benötigte Konzentration kann hierbei (in Abhängigkeit von Form und Größe des zu sterilisierenden Bereiches - im Folgenden steriler Bereich genannt) über einen entsprechend vordefinierten Ausstoß(-menge) an Plasma in den Bereich erreicht werden.

Selbstverständlich ist es aber auch denkbar, eine ausreichende bzw. gewünschte Konzentration an Plasma mit einem entsprechend geeigneten Sensor zu detektieren und vorzugsweise erst bei Erreichen der gewünschten vordefinierten Konzentration den sterilen Bereich für eine Behandlung freizugeben und/oder bei einem Unterschreiten der Konzentration ein Alarmsignal auszugeben. Zudem ist es denkbar, den Ausstoß an Plasma in Abhängigkeit (beispielsweise in Form einer Schwellwert- oder Schwellbereichsregelung) hiervon zu steuern, um eine gewünschte Konzentration oberhalb eines Schwellwertes oder innerhalb eines Bereiches während einer Behandlung zu gewährleisten. Hierbei kann auch bei einem Überschreiten eines Ozon-Grenzwertes, insbesondere durch Verwirbelung im Plasmafeld (z.B. ausgelöst durch zu starke Bewegungen eines Operateurs im sterilen Bereich) im unmittelbaren Umfeld des sterilen Bereiches, beispielsweise über 0,1 ppm, ein Abschalten der Plasmaerzeugung vorgesehen sein.

Das Gehäuse kann hierbei beispielsweise U-förmig, als Polygon oder in Form einer beliebig gekrümmten oder geraden Schiene ausgebildet sein und den Bereich bzw. das Feld zumindest teilweise (offen oder umlaufend) umgeben. Selbstverständlich ist es auch denkbar, einen Teilbereich des Gehäuses schwenk- oder steckbar auszubilden, so dass auch ein Öffnen und Verschließen des Gehäuses ermöglicht wird. Weiterhin ist es denkbar, das Gehäuse in Form eines Gitters auszubilden, so dass auch Stege mit Plasmageneratoren innerhalb des Bereiches liegen können.

In einer Ausgestaltung der Erfindung ist das Gehäuse ringförmig (beispielsweise in Form eines Hohlrings) geschlossen, wobei hierfür neben einem Kreisring beliebige Ringformen wie Polygon, Oval etc, in Frage kommen.

In weiterer Ausgestaltung der Erfindung kann das Gehäuse in Form und Abmessung veränderbar ausgebildet sein. Hierzu ist es denkbar, das Gehäuse in Form von Modulen auszubilden, welche je nach Bedarf, beispielsweise mittels Ineinanderstecken, miteinander verbunden werden können. Selbstverständlich ist es aber auch denkbar, einzelne Elemente des Gehäuses auszieh- und einschiebbar, beispielsweise teleskopartig, auszubilden und auf diese Weise das Gehäuse in seinen Abmessungen und seiner Form zu verändern, um ein gewünschtes Feld zu erzeugen. Die Plasmageneratoren können hierzu in gewünschter Anzahl in im Gehäuse vorhandene Ausnehmungen eingesetzt werden oder bereits im Gehäuse fest eingebaut vorhanden sein.

Nach der Erfindung umfasst die Vorrichtung eine Absaugeinrichtung, um ein stabiles homogenes Feld oder gar eine laminare Strömung zu erzeugen. Hierzu sind im Gehäuse im Wesentlichen gegenüberliegend zu den Plasmageneratordüsen Absaugdüsen vorgesehen.

Für eine Operation, Untersuchung oder beliebige Behandlung wird die Vorrichtung erfindungsgemäß nahe an dem Objekt, insbesondere an der Person, angeordnet oder auch unmittelbar (Kontakt zur Objektoberfläche, insbesondere zur Haut) aufgelegt, so dass der erzeugte sterile Bereich in der Fläche in Gänze das gewünschte OP-Feld abdeckt. Das OP-Feld erstreckt sich hierbei in der Regel auf nur einen Teilbereich der Körperoberfläche, in welchem behandelt und/oder eine Operation durch eine Öffnung in das Körperinnere durchgeführt wird. Aufgrund einer Felddicke von wenigstens einigen Millimetern oder Zentimetern kann hierdurch ein steriles Feld selbst für eine gewünschte Operation gewährleistet werden, bzw. dieser Bereich sterilisiert werden.

Wird die Vorrichtung bzw. das hierdurch erzeugte Plasmafeld - vorzugsweise in einer Ebene parallel zum jeweiligen Körperoberflächenbereich - in unmittelbarem Kontakt zur Haut angeordnet oder ein etwaiger Zwischenraum zwischen Feld und Haut mit einer gegen die Umgebung dichten, sterilen Abdeckung, beispielsweise Folie, Decke etc. abgedeckt, kann selbst während eines invasiven Eingriffs die Sterilität einer Operationsöffnung aufrechterhalten werden. Ein Eindringen von Verunreinigungen, insbesondere Bakterien, in das ungeschützte Körperinnere kann hierdurch auf einfache Weise verhindert werden.

Als Plasmagenerator bzw. Plasmageneratoren kommen hierbei Generatoren zum Einsatz, welche einen Plasmastrahl eines Plasmas bzw. Plasmagases - Im Folgendenden Plasmagas genannt - erzeugen, das (nach Erzeugung und Austritt aus einer Düse des Plasmagenerators) eine Temperatur von kleiner oder gleich 40° C - sogenanntes kaltes Plasmagas - besitzt. Ein für eine Behandlung eines Menschen zu heißes Plasma bzw. ein Plasmastrahl wird hierbei durch geeignete Kühlmaßnahmen (Kühlschlangen, Kühlrippen, Zusatz von kühlem Gas, etc.) vor Austritt aus einer Düse entsprechend gekühlt.

Aufgrund der nahen oder gar unmittelbaren Anwendung und entsprechender Anordnung von Plasmageneratoren an dem Bereich, ist es möglich, StandardPlasmageneratoren zur Erzeugung von kaltem Plasma zu verwenden, welches vorzugsweise zugleich Atmosphärendruck (Atmosphärendruckplasma) oder einen höheren Druck (Hochdruckplasma) besitzen kann.

Deren Strahl bzw. Strömung von Plasmagas - erzeugt vorzugsweise aus Luft, insbesondere Umgebungsluft, eventuell unter Zusatz von Trägergas (beispielsweise Argon) zur Erhöhung der Reichweite - kann in einem (im Vergleich zum sterilen Feld kleinen) Bereich einen antiseptischen Zustand (vorzugsweise höchster Güte) erzeugen, wobei aufgrund der geringen notwendigen Ausstoßrate und/oder der laminaren Strömung (mit Absaugung) eine allenfalls geringe, gesundheitsunschädliche Menge an Ozon (ozonarmes Plasmagas) auftritt. Ein auf diese Weise erzeugter steriler Bereich oder steriles Feld ist daher für Mensch, Tier oder empfindliche Oberflächen von Gegenständen weder gefährlich noch (gesundheits-)schädlich.

Zudem kann durch die Begrenzung des sterilen Feldes auf einen wesentlich kleineren Bereich als eine Körperseitenfläche einer Person vorteilhafterweise auch ein Plasmagas verwendet werden, welches sich in größerer Ausbreitung und damit höherer Konzentration im abgeschlossenen Raum gesundheitsgefährdend auswirken könnte (Ozonkonzentration, Druck, Temperatur, etc.).

Bezüglich der Art der Plasmagasgeneratoren kommen daher alle Plasmaherstellungsarten, insbesondere nicht-thermisches Plasma, in Frage, bei welchen sich zumindest bei einer derart räumlich begrenzten Anwendung eine Gesundheitsgefährdung (zu hohe Ozonkonzentration, zu starke Hitze, zu hoher Druck etc.) für eine Person oder einen Patienten vermeiden lässt.

Zudem ist es zur Verminderung einer Gesundheitsgefährdung denkbar, eine unerwünscht hohe Ozonkonzentration - durch Einsatz geeigneter Filter, wie HEPA-Filter (High Efficiency Particulate Airfilter), ULPA-Filter (Ultra Low Penetration Air) und SULPA-Filter (Super ULPA) etc., im Plasma bzw. dem Plasmastrahl - zu verringern oder gar zu vermeiden. Diese Filter können auch in den Gasstromzufuhr (insbesondere Luft) zu den Plasmageneratoren (vor Erzeugung des Plasmas) eingesetzt werden, um ein Ansaugen von Staub, Partikeln oder Mikroorganismen zu verhindern.

Durch den Einsatz mehrerer Plasmageneratoren können vorteilhafterweise auch Generatoren verwendet werden, welche nur einen Plasmastrahl bzw. -bereich geringerer Tiefe erzeugen, da ein Felddurchmesser mit zwei gegenüberliegenden Generatoren halber Plasmafeldtiefe (Durchmesser/2) abgedeckt werden kann.

In bevorzugter Ausgestaltung der Erfindung wird der sterile Bereich durch den Einsatz von mehreren (wenigstens 2, 3, 4, 5, 6 oder mehr), insbesondere Plasmageneratoren erzeugt, welche in einem das Feld bzw. den Bereich zumindest teilweise umgebenden offenen oder geschlossenen Gehäuse derart angeordnet sind, dass Plasma bzw. Plasmagas in das Feldinnere, vorzugsweise im Wesentlichen innerhalb derselben Ebene (koplanar) emittiert wird. Selbstverständlich ist es aufgrund der Begrenzung des OP-Feldes nach unten denkbar, einzelne oder alle Plasmageneratoren bzw. deren Austrittsdüsen leicht nach unten geneigt anzuordnen oder schwenkbar auszubilden, so dass etwaige Vertiefungen im OP-Feld leichter und schneller erfasst werden.

Durch die Verwendung von mehreren Plasmageneratoren ist es auf einfache und kostengünstige Weise möglich, einen Bereich bzw. ein Feld mit sterilisierender, insbesondere medizinisch (dauer-)sterilisierender, Wirkung zu erzeugen. Als Plasmageneratoren können vorteilhafterweise auch Standardplasmageneratoren verwendet werden, die einen Strahl bzw. eine Strömung von kaltem Plasma(gas) - vorzugsweise unter Verwendung von Luft, insbesondere Umgebungsluft, erzeugen.

Ein derartiges Feld O kann beispielsweise eine Fläche von einigen Zentimetern Durchmesser oder Schenkelabstand, beispielsweise 10 bis 60 cm, insbesondere 20 bis 30 cm, vorzugsweise mindestens 25 cm oder mindestens 30 cm, umfassen. Für das Erzeugen eines Feldes O mit einem größeren (je nach Form des Feldes mittleren oder maximalen) Durchmesser, beispielsweise im Wesentlichen größer als 50 cm oder 60 cm, kann ein zusätzliches Trägergas (insbesondere Argon) - beispielsweise mittels einer Gebläseeinrichtung - zugesetzt werden, um die Reichweite eines Plasmastrahls eines Plasmagenerators zu erhöhen.

In seiner Tiefe bzw. Dicke kann das Feld bzw. der Bereich eine gewünschte sterilisierende Wirkung und damit entsprechende Intensität innerhalb von (einem bis) wenigen Millimetern bis zu mehreren Zentimetern, beispielsweise 5 bis 20 cm, vorzugsweise 1 bis 10 mm (laminare Strömung), aufweisen.

In besonders bevorzugter Ausgestaltung der Erfindung wird während eines operativen Eingriffs oder einer Behandlung vorzugsweise von oben bzw. außen durch das erzeugte sterile Feld hindurch in Richtung des Körpers einer zu behandelnden Person agiert, so dass vorteilhafterweise auch OP-Instrumente und Hände bzw. Handschuhe während des Durchdringens des Feldes (erneut) sterilisiert werden.

Das sterile Feld bietet in dieser medizinischen Anwendung im Nahbereich einer Person selbst bei einem invasiven Eingriff und damit Durchbrechen der schützenden Hautschichten einen sicheren Schutz gegen ein Eindringen von unerwünschten nicht sterilen Partikeln, wie lebende Organismen, Sporen, Keime, Viren, insbesondere multiresistente Bakterien. Das bei Behandlungen, insbesondere Operationen, bestehende Infektionsrisiko kann hierdurch deutlich verringert werden.

Mit der erfindungsgemäßen Vorrichtung kann der Restgehalt an vermehrungsfähigen Mikroorganismen im sterilen Feld auf 10⁻⁷ oder gar 10⁻⁸ Kolonie bildende Einheiten reduziert werden und liegt damit deutlich unter dem geforderten Wert von 10⁻⁶.

In weiterer Ausgestaltung der Erfindung weist die Vorrichtung einen, beispielsweise mit Hilfe von Gelenken, beweglichen Arm auf, an welchem das Gehäuse, vorzugsweise um eine Achse in der Feldebene drehbar, angeordnet ist. Mittels dieses Arms bzw. dieser Halterung kann die Vorrichtung vertikal und/oder horizontal und/oder axial in beliebige Positionen gebracht werden, so dass unterschiedlichste OP-Felder abgedeckt werden können.

In weiterer Ausgestaltung der Erfindung kann das Feld eine variable Größe und/oder Form aufweisen. Hierzu können beispielsweise entsprechend unterschiedlich ausgebildete, vorzugsweise am Arm austauschbare Gehäuse vorgesehen sein, in welche eine entsprechende Anzahl von Plasmageneratoren eingesetzt wird oder bereits fest installiert ist. Selbstverständlich ist es aber auch denkbar, das Gehäuse selbst in seinen Abmessungen veränderbar, beispielsweise schiebbar, steckbar etc. auszubilden, wobei je nach eingestellter Größe auch eine entsprechend unterschiedliche Anzahl von Aufnahmen für Plasmageneratoren vorgesehen sein kann.

In einer anderen Anwendung der vorstehend erläuterten Vorrichtung kann die Vorrichtung verwendet werden, um Personen beispielsweise in einer Schleuse zu sterilisieren. Hierzu wird die Vorrichtung insgesamt, beispielsweise in Form eines Scan-Vorgangs, nach und nach über den gesamten Körper, beispielsweise von oben nach unten oder umgekehrt geführt, so dass das Feld nach und nach jeden (Außen-)Bereich einer Person sterilisiert. Hierbei kann das Feld beispielsweise senkrecht zur Körperachse einer Person durch Bewegen bzw. Verfahren (Scannen) der Vorrichtung entlanggeführt werden.

In vorteilhafter Ausgestaltung der Erfindung kann das Gehäuse als umlaufender Ring beliebiger Form, insbesondere Kreisring, eventuell mit einem zu öffnenden und verschließbaren Teilbereich, ausgebildet sein.

Weitere vorteilhafte Ausgestaltungen der Erfindung ergeben sich aus den Unteransprüchen.

Die Erfindung wird nachfolgend anhand eines in der Zeichnung dargestellten Ausführungsbeispiels näher erläutert. In der Zeichnung zeigen:
- Fig. 1: eine schematische Seitenansicht eines abgeschlossenen Raums mit einer erfindungsgemäßen Vorrichtung zur Sterilisation eines Operationsbereichs;
- Fig. 2: eine schematische Ansicht (Grundriss) eines abgeschlossenen Raums in Form einer Schleuse mit einer erfindungsgemäßen Vorrichtung zur Sterilisation einer darin befindlichen Person und
- Fig. 3: eine schematische Seitenansicht eines abgeschlossenen Raums mit einer Abwandlung der erfindungsgemäßen Vorrichtung nach Fig. 1.

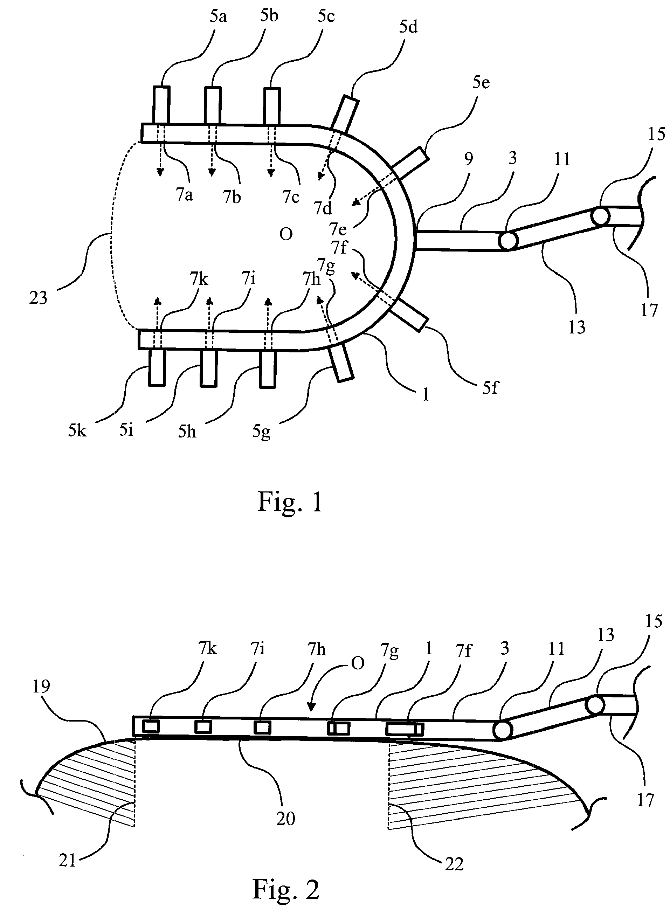

Die in Fig. 1 dargestellte Vorrichtung umfasst ein U-förmiges Gehäuse bzw. eine Halterung 1, welche über eine vorzugsweise gelenkige Verbindung 9 mit einem ersten Teilbereich 3 eines Haltearms in der Längsachse diese Teilbereiches 3 drehbar und/oder in horizontaler und/oder vertikaler Ebene schwenkbar angeordnet ist. An diesen Teilbereich 3 schließt sich über ein erstes Gelenk 11 ein zweiter Teilbereich 13 und an diesen wiederum über ein zweites Gelenk 15 ein dritter Teilbereich 17 an. Die Gelenke 11 und 13 können hierbei innerhalb der Zeichenebene und/oder einer hierzu senkrechten Ebene dreh- oder schwenkbar ausgebildet sein. Je nach Anforderung kann der Haltearm auch aus weniger oder mehr Teilbereichen ausgeführt sein, um eine erforderliche Position zu erreichen.

Auf diese Weise kann nicht nur die Vorrichtung insgesamt (beispielsweise auf Rollen oder Rädern) durch eine Person im Raum an eine gewünschte Position geschoben werden, sondern das Gehäuse 1 und damit das sterile Feld O in seiner Richtung frei in allen Freiheitsgraden ausgerichtet werden.

Der Haltearm kann Teil eines Stativs sein, welches am Boden fest verankert ist oder beispielsweise mittels Rollen bewegt werden kann. Hierbei kann die Vorrichtung stationär oder auch autark (Koffer, zum Aufbauen) mit autarker Energieversorgung (Akku) ausgebildet sein.

Selbstverständlich ist es auch denkbar, den Haltearm an seinem einen Ende fest an einer Wand zu verankern.

Auf diese Weise kann das Gehäuse komfortabel in eine gewünschte Position gebracht werden, wobei die Gelenke derart ausgebildet sind, dass die Vorrichtung in einer beispielsweise von Hand eingestellten Position - ähnlich einer schwenkbaren Schreibtischlampe - verharrt oder zusätzlich fixiert werden kann.

Anschlüsse für die Plasmageneratoren 5a bis 5k und eine etwaige Absaugvorrichtung sowie eine optional zusätzliche Versorgung mit Luft/Gas oder Argon der Plasmageneratoren können von außen in in der Zeichnung nicht näher dargestellter Weise an die Plasmageneratoren herangeführt sein. Selbstverständlich ist es aber auch denkbar, entsprechende Versorgungszuführungen oder -leitungen innerhalb des Gehäuses 1 (Hohlraumbauweise) vorzusehen.

Fig. 2 zeigt die Vorrichtung bzw. das Gehäuse 1 in einer Position über einer Bauchdecke 19 einer senkrecht zur Zeichenebene (beispielsweise auf einem OPTisch) liegenden Person bzw. eines Patienten. Wie in Fig. 2 dargestellt, ist das Gehäuse unmittelbar auf der Bauchdecke 19 aufgelegt (unmittelbarer Kontakt zur Hautoberfläche) oder nahe darüber angeordnet. Das Gehäuse 1 weist hierbei im Inneren ein Plasmafeld O auf, welches auch an der offenen Seite einen als gestrichelte Linie dargestellten Feldrand 23 aufweist.

Ein Arzt kann hierbei von oben durch das Plasmafeld O hindurch die Bauchdecke 19 behandeln, oder die Bauchdecke 19 gar öffnen, ohne dass hierbei Keime an die Bauchdecke 19 gelangen oder durch diese in das Körperinnere eindringen. Hierbei werden zudem Hände, Handschuhe, Geräte, etc., welche in das Feld O eindringen, ebenfalls (nach einer üblicherweise erfolgten Sterilisation erneut) sterilisiert, so dass nicht nur ein Eindringen von Keimen aus der Umgebung (Luft), sondern auch durch einen Operateur selbst verhindert wird. Das Plasmafeld O weist hierbei eine gewisse Dicke in der Mitte (Bauchbildung) von beispielsweise einigen Zentimetern, insbesondere 2 bis 5 cm oder bis zu 10 cm, vorzugsweise 0,5 bis 2 cm, mit einer minimalen Dicke (an den Feldseiten) von wenigstens 1 mm oder 1 cm auf.

Zusätzlich kann um das Gehäuse 1 nach außen abdeckend oder gar abdichtend eine nicht näher dargestellte Abdeckung in Form einer Folie oder eines Tuches vorgesehen sein, um den Bereich außerhalb eines zu behandelnden Bereiches, insbesondere OP-Bereiches 20, abzudecken oder gar den Operationsbereich 20 bzw. das Äußere des Gehäuses 1 gegenüber der Bauchdecke 19 abzudichten. Im Falle einer Bauch-Operation kann hierdurch ein Operationsbereich 20 und eine Öffnung im Bauchraum, welche in Fig. 2 schematisch durch gestrichelte Linien 21, 22 dargestellt ist, keimfrei gehalten werden und gleichzeitig durch das den Operationsbereich 20 abdeckende Plasmafeld O hindurch operiert werden.

Wie aus Fig. 1 und Fig. 2 ersichtlich, befinden sich an dem Gehäuse Plasmageneratoren 5a bis 5k, welche in Ausnehmungen 7a bis 7k des Gehäuses 1 angeordnet sind und nach innen in Pfeilrichtung Plasmagas in das Feld O emittieren.

In gegenüberliegenden Schenkeln des U-förmigen Gehäuses 1 sind hierbei Plasmageneratoren 5a, 5b, 5c zu den Plasmageneratoren 5k, 5i und 5h einander gegenüberliegend angeordnet, so dass die Tiefe des jeweiligen Plasma(gas)strahls bis zur Feldmitte in ausreichender Konzentration reicht, um ein Plasmafeld mit einer ausreichenden sterilisierenden Wirkung bzw. ausreichender Konzentration an Plasma, insbesondere für eine Operation (beispielsweise Bauchraum-OP) zu erzeugen.

Die Plasmageneratoren 5a bis 5k emittieren hierbei vorzugsweise Plasmagas innerhalb derselben Ebene des Feldes O (in Fig. 2 senkrecht zur Zeichenebene), wobei eine derartige gerichtete Einströmung auf einfache Weise durch entsprechende Ausrichtung einer jeden Düse eines Plasmagenerators 5a bis 5k erreicht werden kann. Die Düsen können hierbei auch durch die Ausnehmungen 7a bis 7k hindurchreichen oder durch diese selbst ausgebildet sein. Um Vertiefungen im OP-Feld bzw. Feld O besser und schneller zu erfassen können, die Plasmageneratoren bzw. deren Düsen auch leicht nach unten geneigt sein oder bei Bedarf geschwenkt (mittels einer Schwenkvorrichtung) werden. Aufgrund eines Absinkens des erzeugten Plasmagases werden aber auch bei einem koplanaren Einströmen Vertiefungen im Feld O schnell und sicher mit Plasmagas benetzt.

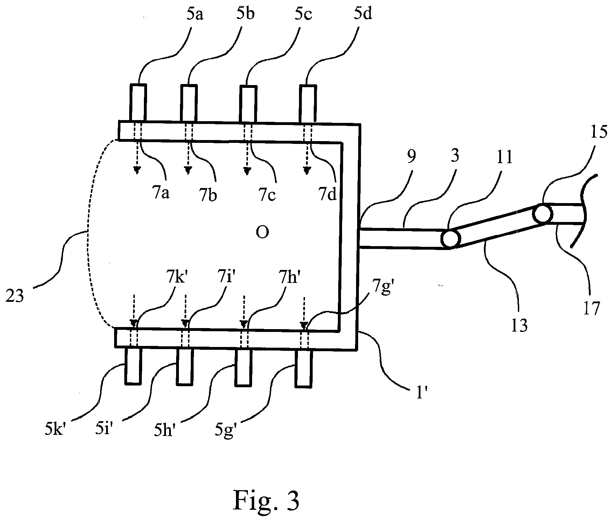

Wie in Fig. 3 dargestellt, ist es auch denkbar, anstelle gegenüberliegender Plasmageneratoren 5a, 5b, 5c zu 5k, 5i und 5h, gegenüberliegend zu den Plasmageneratoren 5a, 5b, 5c wenigstens eine Absaugeinrichtung 5k', 5i', 5h', 5g' mit Absaugdüsen 7k', 7i', 7h', 7g' (beispielsweise anstelle der Plasmageneratoren 5k, 5i, 5h, 5g) oder vice versa anzuordnen. Als Gehäuseform ist in Fig. 3 ein an der Vorderseite offenes rechteckiges Gehäuse 1' dargestellt, wobei selbstverständlich auch andere Grundformen möglich sind. Die weiteren Elemente in Fig. 3 entsprechen den bereits in Fig. 1 beschriebenen Elementen, so dass diesbezüglich identische Bezugsziffern verwendet wurden.

Einzelne Plasmageneratoren, wie beispielsweise in Fig. 3 anhand der Positionen 5e-5f dargestellt, können auch - je nach Geometrie des Feldes O - in unterschiedlichen Ausführungsformen entfallen, da Plasmagas in ausreichender Weise bereits durch Plasmageneratoren 5a-5d, (und evtl. 5k, 5i, 5h, 5g) auf einer oder auf beiden, vorzugsweise geraden, Schenkelseite(-n) erzeugt werden kann. Das Absaugen kann hierbei auch durch eine einzige in der Zeichnung nicht dargestellte Absaugeinrichtung erfolgen, welche alle Düsen 7g', 7h', 7i', 7k' mit einem entsprechenden Unterdruck versorgt. Die Anschlüsse (beispielsweise Schläuche) hierfür können außerhalb des Gehäuses 1 zur gemeinsamen Absaugeinrichtung geführt werden. Selbstverständlich ist es aber auch denkbar, als Verbindung zwischen Düsen 7g' 7h', 7i', 7k' und Absaugeinrichtung eine Hohlraumführung, beispielweise einen als Hohlrohr ausgebildeten Schenkel, in dem Gehäuse 1 vorzusehen. Selbstverständlich ist es auch in allen Ausführungsformen denkbar, das Plasmagas durch eine Hohlraumführung, beispielweise durch einen als Hohlrohr ausgebildeten Schenkel, zu den Düsen zuzuführen. In diesem Fall kann wenigstens ein Plasmagenerator auch an anderer als in der Zeichnung dargestellter Stelle angeordnet sein.

In allen Ausgestaltungen der Erfindung kann vorteilhafterweise ein stabiles, homogenes Feld oder gar eine laminare Strömung erzeugt werden. Hierbei kann eine Flussrate des Plasmagases von einigen Metern pro Sekunde, vorzugsweise 2-10m/s, insbesondere 4-5 m/s, von der Austrittsdüse eines Plasmagenerators 5a, 5b, 5c infolge der laminaren Strömung im Wesentlichen über das gesamte Feld O aufrechterhalten werden. Vorteilhafterweise ergibt sich hierdurch ein besonders homoges Feld O (Vermeidung von Wirbelbildung), ohne dass ein Operateur hierdurch gestört wird oder ein OP-Bereich in merklicher Weise austrocknet. Für die Erzeugung einer laminaren Strömung emittieren Plasmageneratoren 5a, 5b, 5c bzw. deren Düsen vorzugsweise parallel zueinander (insbesondere koplanar oder tangential zur behandelnden Oberfläche) Plasmagas in das Feld O. Selbstverständlich können auch 4, 5, 6, 7, 8 oder mehr Generatoren hierzu auf einem Schenkel angeordnet sein und vorzugsweise bei Bedarf zugeschaltet werden.

In hierzu komplementärer Weise sind gegenüberliegende (parallele) Absaugdüsen 7g', 7h', 7i', 7k' am anderen Schenkel (insbesondere koplanar oder tangential zur behandelnden Oberfläche) angeordnet, welche das Plasmagas in nicht näher dargestellter Weise abführen (entfernt vom Feld O ausstoßen, entionisieren, filtern, insbesondere mittels Aktivkohlefilter oder Rekombinationsfilter, temporär speichern, Gas bzw. Luft in nicht ionisiertem Zustand ausstoßen). Insbesondere in einem Feld O mit laminarer Plasmagasströmung werden auch Vertiefungen im Feld O erfasst bzw. von (absinkendem) Plasmagas benetzt. Ebenso werden Erhöhungen durch die Strömung miterfasst. Die laminare Strömung folgt dabei Tälern und Erhöhungen im Feld O.

Das Plasmafeld O kann - in beliebiger Ausgestaltung der Erfindung - je nach Anforderung eine Feldgröße mit einem mittleren Durchmesser (insbesondere Schenkelabstand) von 10 cm bis 60 cm, vorzugsweise bis 30 cm oder 40 cm besitzen.

Um unterschiedliche Größen und/oder Formen zu realisieren, ist es zudem auch denkbar, die Vorrichtung, insbesondere das Gehäuse 1 selbst veränderbar, beispielsweise ausziehbar, steckbar etc. auszubilden. Beispielsweise ist es denkbar, die in Fig. 1 dargestellten geraden Schenkelbereiche des Gehäuses 1 teleskopartig ausziehbar und einschiebbar auszubilden, wobei im verlängerten Zustand im Vergleich zum eingeschobenen (verkürzten) Zustand zusätzliche Ausnehmungen vorgesehen sein können, in welche bei Bedarf weitere Plasmageneratoren eingesetzt werden können, um in das vergrößerte Feld O Plasma mit für eine Sterilisation ausreichender Konzentration zu erzeugen.

In beliebiger Ausgestaltung der Erfindung kann, je nach Anzahl vorhandener Mikroorganismen, nach einigen Sekunden oder mehreren Minuten, beispielsweise 20 s bis 15 min, eine gewünschte Sterilität (Restgehalt an vermehrungsfähigen Mikroorganismen) im sterilen Feld O von 10⁻⁶, vorzugsweise unter 10⁻⁷ oder gar unter 10⁻⁸, Kolonie bildenden Einheiten erreicht werden.

Ozon wird hierbei selbst in unmittelbarer Nähe des Feldes O nur in geringem Maße in einer Konzentration unter 0,1 ppm, vorzugsweise unter 0,5 oder unter 0,3, insbesondere von 0,028ppm, gebildet. Im Feld O herrscht in den unterschiedlichen Ausgestaltungen der Erfindung eine Konzentration positiv geladener Elektronen von 10⁹ bis 10¹².

In besonderer Ausgestaltung der Erfindung wird nur Umgebungsluft für die Erzeugung des Plasmagases verwendet, so dass auf die Zufuhr von zusätzlichem (Träger-)Gas, insbesondere Argon, verzichtet werden kann.

Das Feld O wird in seiner vorgegebenen Größe (beispielsweise einer Fläche von wenigstens 400, 600, 700, 1000 oder 1500 cm²) insgesamt von Plasma bzw. Plasmagas in einer (durchschnittlichen) Dicke von einigen Zentimetern, insbesondere von wenigstens einigen Millimetern, 1 cm, 2 cm, 3cm, oder mehr ausgefüllt. Bei einem Feld O mit vorzugsweise laminarer Strömung ist es auch möglich, ein kontinuierliches Feld mit geringer Dicke von seitlich wenigstens 1 mm bis zur Mitte (Bauchbildung bei einer laminaren Strömung in der Mitte bzw. Verjüngung in Richtung Düsen) mit maximal 10-25 mm bei einer Fläche von 30cm x 30cm zu erzeugen, ohne dass die Strömung abreißt und nicht sterile Bereiche innerhalb des Feldes entstehen können.

Wie vorstehend beschrieben kann durch die Plasmageneratoren 5a bis 5k bei Bedarf ein sterilisierendes Plasmafeld O erzeugt werden. Das Emittieren von Plasma(gas) in das Feld O bzw. den sterilen Bereich kann hierbei von den Plasmageneratoren impulsartig oder kontinuierlich erfolgen. Eine ausreichende Konzentration an Plasma im Feld kann beispielsweise zusätzlich mittels eines Sensors überwacht und gegebenenfalls für spätere Zwecke protokolliert werden. Ein derartiger Sensor kann beispielsweise am Gehäuse direkt oder über einen Arm in das Feld O einschwenkbar vorgesehen sein. Hierbei kann bei einer bekannten Verteilung von Plasma im Feld O der Sensor an beliebiger Stelle innerhalb des Feldes O angeordnet sein, so dass bei bekannter Verteilung ein zu erreichender Mindestschwellwert am Ort des Sensors voreingestellt werden kann, um an beliebiger Stelle des Feldes O ein Erreichen einer ausreichenden Konzentration an Plasma zu detektieren oder gar ein Überwachen der Konzentration zu gewährleisten.

Durch die erfindungsgemäße Vorrichtung wird vorteilhafterweise auch eine Resterilisierung von Handschuhen und Instrumenten erreicht, welche in Berührung mit dem Feld O kommen, in dieses eindringen, oder dieses durchdringen. Hierdurch kann ein Eindringen von Keimen, Bakterien, Pilzen, Sporen, in den Behandlungsbereich, insbesondere die OP-Öffnung, verhindert werden. Zudem können Instrumente (insgesamt, selbst an der aufliegenden Seite) in einem für eine Operation nicht benötigten Bereich des Feldes O abgelegt und dort steril gehalten werden. Hierzu können Stege, Schalen, Netze o.ä. vorgesehen sein, welche vorzugsweise bei Bedarf, beispielsweise angelenkt an der Halterung, in das Feld O eingeschwenkt oder angeordnet (aufgesetzt, aufgesteckt, etc.) werden können.

Selbstverständlich ist die Erfindung nicht auf das dargestellte Ausführungsbeispiel begrenzt. Beispielsweise kann die Erfindung auch verwendet werden, um eine (Zugangs-)Schleuse für Personen mit Plasmasterilisation zu realisieren. Hierzu kann vorzugsweise ein umlaufendes ringförmiges Gehäuse von oben und/oder unten mit einem Plasmafeld O senkrecht zur Körperachse entlang einer Person oder eines Objekts verfahren werden, um die Person oder das Objekt zu sterilisieren. Eine derartige Schleuse kann beispielsweise vor einem Eingang von Räumen (OP-Raum) oder Gebäuden (Kliniken) Anwendung finden, in welchen bereits eine zumindest weitestgehend keimfreie Umgebung herrscht oder eine weitere Verunreinigung mit externen Keimen verhindert werden soll. So ist es auch denkbar, derartige Schleusen vor öffentlichen Gebäuden mit hoher Besuchsrate und damit hohem Infektionsrisiko (Bahnhöfe, Ämter, Geschäfte, etc.) oder auch für private Häuser und Wohnungen zur Vermeidung von externen Verunreinigungen und daraus resultierender Infektionsgefahr zu verwenden.

### Bezugszeichenliste

- O: Plasmafeld
- 1, 1': U-förmiges Gehäuse/Halterung
- 3: Haltearm erster Bereich
- 5a-5k: Plasmageneratoren
- 7a-7k: Ausnehmungen/Duchgangsöffnungen
- 5g'-5k': Absaugeinrichtungen
- 7g'-7k': Absaugdüsen
- 9: Verbindung zwischen Halterung 1 und Arm 3
- 11: erstes Gelenk
- 13: Haltearm zweiter Bereich
- 15: zweites Gelenk
- 17: Haltearm dritter Bereich
- 19: Bauchdecke
- 20: zu behandelnder/operierender Teilbereich der Bauchdecke 19
- 21: Grenze (muss nicht gerade sein) steriler Bereich/Operationsbereich (in die Tiefe geschützt vor einem Eindringen von Keimen/Bakterien)
- 22: Grenze (muss nicht gerade sein) steriler Bereich/Operationsbereich (in die Tiefe geschützt vor einem Eindringen von Keimen/Bakterien)
- 23: vordere Begrenzung des Plasmafeldes O

## Patentansprüche

1. Vorrichtung zur Erzeugung eines sterilen Bereiches (O) für eine Operation, Untersuchung oder Behandlung wenigstens eines Teilbereiches (20) eines Objekts (19), insbesondere einer Person, wobei die Vorrichtung wenigstens einen Plasmagenerator (5a-5k) aufweist, mittels welchem bei Bedarf kaltes Plasmagas in den Bereich (O) emittiert wird und die Vorrichtung eine Absaugeinrichtung in Form von im Wesentlichen gegenüberliegend zu dem wenigstens einen Plasmagenerator (5a-5k) vorgesehene wenigstens eine Absaugdüse (7k'-7g') umfasst, um ein stabiles homogenes Feld oder gar eine laminare Strömung zu erzeugen.

2. Vorrichtung nach Anspruch 1, **dadurch gekennzeichnet, dass** die Vorrichtung am Rand des Bereichs (O) ein zumindest teilweise umgebendes Gehäuse (1) aufweist, in welchem mehrere Plasmageneratoren (5a-5k) derart angeordnet sind, dass sie bei Bedarf Plasmagas in Richtung zum Inneren des Bereiches (O) emittieren.

3. Vorrichtung nach Anspruch 2, **dadurch gekennzeichnet, dass** die mehreren Plasmageneratoren (5a-5k) derart angeordnet sind, dass sie bei Bedarf ein planares, steriles Feld erzeugen.

4. Vorrichtung nach einem der vorhergehenden Ansprüche, **dadurch gekennzeichnet, dass** das Gehäuse (1) U-förmig ausgebildet ist.

5. Vorrichtung nach einem der Ansprüche 1 bis 3, **dadurch gekennzeichnet, dass** das Gehäuse (1) ringförmig geschlossen ausgebildet ist.

6. Vorrichtung nach einem der vorhergehenden Ansprüche, **dadurch gekennzeichnet, dass** die Vorrichtung ein in Form und Abmessungen veränderbares Gehäuse (1) aufweist.

## Claims

1. A device for generating a sterile area (O) for an operation, examination or treatment of at least one partial area (20) of an object (19), in particular of a person, wherein the device has at least one plasma generator (5a-5k), by means of which, as needed, cold plasma gas is emitted into the area (O), and the device comprises a suction device in the form of at least one suction nozzle (7k'-7g'), provided substantially opposite the at least one plasma generator (5a-5k), in order to generate a stable homogeneous field or even a laminar flow.

2. A device according to claim 1, **characterised in that** the device has, at the edge of the area (O), an at least partially surrounding housing (1) in which a plurality of plasma generators (5a-5k) are arranged in such a way that, as needed, they emit plasma gas in the direction of the interior of the area (O).

3. A device according to claim 2, **characterised in that** the plurality of plasma generators (5a-5k) are arranged in such a way that, as needed, they generate a planar sterile field.

4. A device according to one of the preceding claims, **characterised in that** the housing (1) is formed in a U-shaped manner.

5. A device according to one of claims 1 to 3, **characterised in that** the housing (1) is formed so as to be closed in a ring-shaped manner.

6. A device according to one of the preceding claims, **characterised in that** the device has a housing (1) whose shape and dimensions can be changed.

## Revendications

1. Dispositif permettant de créer une zone stérile (O) en vue d'une intervention chirurgicale, d'un examen ou d'un traitement d'au moins une zone partielle (20) d'un objet (19), en particulier d'une personne, dans lequel le dispositif présente au moins un générateur de plasma (5a-5k), au moyen duquel, si besoin, du gaz de plasma froid est émis dans la zone (O) et le dispositif comprend un système d'aspiration sous la forme d'au moins une buse d'aspiration (7k'-7g') prévue sensiblement du côté opposé à l'au moins un générateur de plasma (5a-5k) pour produire un champ homogène stable ou même un courant laminaire.

2. Dispositif selon la revendication 1, **caractérisé en ce que** le dispositif présente sur le bord de la zone (O) un boîtier (1) au moins partiellement périphérique dans lequel plusieurs générateurs de plasma (5a-5k) sont disposés de telle sorte qu'ils émettent si besoin du gaz de plasma en direction de l'intérieur de la zone (O).

3. Dispositif selon la revendication 2, **caractérisé en ce que** les plusieurs générateurs de plasma (5a-5k) sont disposés de telle sorte qu'ils produisent si besoin un champ planaire stérile.

4. Dispositif selon l'une quelconque des revendications précédentes, **caractérisé en ce que** le boîtier (1) est formé en forme de U.

5. Dispositif selon l'une quelconque des revendications 1 à 3, **caractérisé en ce que** le boîtier (1) est formé en forme d'anneau fermé.

6. Dispositif selon l'une quelconque des revendications précédentes, **caractérisé en ce que** le dispositif présente un boîtier (1) dont la forme et les dimensions peuvent être modifiées.
